# EUROPEAN PATENT APPLICATION

(11) **EP 2 492 672 A2**
(43) Date of publication of application: **29.08.2012**
(21) Application number: 11166886.9
(22) Date of filing: 20.05.2011
(51) Int. Cl.: G01N 25/72, G01N 33/42

(54) **Infrared imaging for road surface defects detection**

(30) Priority: 25.02.2011 CN 201110048227
(71) Applicant: Guangzhou SAT Infrared Technology Co., Ltd., Guangzhou Guangdong 510730 (CN)
(72) Inventor: Wu, Jiping, Guangzhou (CN); Li, Yuenian, Guangzhou (CN)
(74) Representative: Gervasi, Gemma

(57) **Abstract**

A system and method for road surface defects detection based on infrared imaging technology comprising: a detection vehicle (1) traveling on a detected road surface; a pan and tilt (2) provided on the detection vehicle (1) and rotate horizontally and vertically with respect to the detection vehicle (1); an infrared camera (3) detachably set on the pan and tilt (2), which is used to capture infrared thermal images of the detected road surface, and to output inferred thermal image digital signals about the inferred thermal images including temperature values of the detected road surface; a main controller (4) provided on the detection vehicle (1) and connected to the pan and tilt and the infrared camera (3) respectively, which is used to control capture actions of the infrared camera (3), to control angles of the horizontal and vertical rotations of the pan and tilt (2), and to transform the infrared thermal image digital signals output from the infrared camera (3) into digital signals to be used in standard network transmission; and a data processor (5), used to receive the digital infrared signals to be analyzed and processed to determine the type and location of defects on the detected road surface.

## Description

### FIELD OF THE INVENTION

The present invention relates to a system and method for road surface defects detection, especially to a system and method for monitoring the construction quality of the road surface of the bituminous highway and the like.

### BACKGROUND

Conventionally, in China and as well in foreign countries, when monitoring on the surface defects of the bituminous highway and the like, people often use "artificial visual test method" or "evaluation and supporting maintenance technology for water seepage (dank) of the road surface based on infrared thermal differences" to make rapid detection and evaluation on defects on the road surface.

The core device used by the "evaluation and supporting maintenance technology for water seepage of the road surface based on infrared thermal differences" is an infrared thermometer (or an so-called infrared spot thermometer), while the system for road defects detection of which fastens several infrared thermometers in parallel to the tail portion of the detection vehicle. The Infrared thermometer, which is triggered by a distance sensor during the vehicle's traveling, collects temperature values within the monitoring area on road surface of the highway and the like, uses multi-point temperature values to form temperature differences curves, and then uses the temperature differences curves to form thermal differences (temperature differences) graphs of the road surface in simulation as the detection data of the road surface, thus to estimate the damaged degree of the road surface.

The Infrared thermometer is a device using the photoelectric detector installed therein to detect surface temperatures of an object, wherein the photoelectric detector emits infrared beam that is to be reflected from its encountered surface and then gathered on the photoelectric detector. Compared to the aforementioned emitted beam, the infrared thermal energy carried by the returned beam have energy loss to different degrees with respect to the above described object in different materials and surface states, the optical detector receives the energy of the returned infrared beam and convert it to corresponding electrical signal, the signal is then converted to a temperature of the measured target.

Since the infrared thermometer is used to measure temperature of one certain spot on the object's surface once at a time, the ideal status is to gather the emitted beam to one exact spot on the object's surface, so that the measured temperature is the most accurate for that spot. However, due to the scattering effect of the optical beam, the beam emitted from the photoelectric detector is spread to a certain area other than an exact spot when finally reaches the object's surface after passing through a certain distance, while the size of this area is clearly in proportional with the passing distance (i.e. the temperature measuring distance) between the photoelectric detector and the detected object. Therefore, the temperature measured by the infrared thermometer is actually an average temperature of the certain area.

Moreover, although the infrared thermometer can tell temperatures on some spots(or areas) of the detected objects through one or more measurements, the detected objects can hardly provide a visual image for the detected object or provide the infrared thermal image that reflects the structural characteristics of the detected object. Thus the existing method for road defects detection based on the infrared thermometer, since there is no real scene images available as references, obtains its detection results not quite intuitionistic, thus resulting in difficult determination, location and analysis on the road's defects, which is very likely to cause miss detections or error measurements that fail to meet the requirements in actual monitoring and evaluating.

For example, patent application documents of No. CN200420041784.4, in title of "Infrared road surface temperature tester", discloses such a system for road surface defects detection, which uses infrared thermometer to collect temperatures of the bituminous road surface, forms thermal differences graphs according to the temperature differences, and evaluates the dank areas from three aspects of quantitative, qualitative and positioning ones, which has a main principle that is: because of the inconsistency in density and water seepage of the road which results in different after-rain water containing for the structural layer of the road, and the road surface shows up different temperature reflections as the heat is swept away by sunshine evaporation, thus the differences in density can be determined by analyzing the thermal differential graphs.

Since this system uses the infrared thermometer as its core detecting device, and its working principle determines that it can be only used to detect temperatures but not to form images, thus it can not directly reflect the thermal distribution image of the detected object; and because the digital infrared signals measured by the infrared thermometer is an average temperature of a certain area (measured area) which is in proportional with the measurement distance, so the results of the temperature measurement may show up certain errors in accuracy; furthermore, since the measured temperature data of the infrared thermometer is a kind of dotted data (no more than an digital infrared signal of a small area, and in considering of the accuracy of road measurement, this area is supposed not too big), so the detected area of a single infrared thermometer is very limited. Even if there are pluralities of infrared thermometers to be additionally mounted on the detection vehicle, only one lane can be monitored once at a time, so it is certainly difficult and time-consuming to thoroughly measure all lanes in a multi-lane road. For example, in the specification of the above patent application, it says in its illustration on Figure 1 that: "The graph area: have graphs formed in accordance with the thermal differences, the reddish one therein representing high temperature, and the bluish one representing low temperature, and only one lane is detected once at a time", thus it can be known from which that only one single lane can be detected for the above patent application.

### SUMMARY OF THE INVENTION

In order to solve the above problems in the prior art, an object of the present application is to provide a system for road surface defects detection based on the infrared imaging technology, which is to rapidly and visually detect the defects on bituminous road surface of the highway and the like, therefore to provide exact technical basis for maintaining and repairing on water seepage, cracks, notches, looseness, interlayer desquamation and so on.

To achieve the above object, the present application provides a system for road surface defects detection, while the system comprising: a detection vehicle traveling on a detected road surface; a pan and tilt provided on the detection vehicle and rotate horizontally and vertically with respect to the detection vehicle; an infrared camera detachably set on the pan and tilt, which is used to capture infrared thermal images of the detected road surface, and to output inferred thermal image digital signals about the inferred thermal images including temperature values of the detected road surface; a main controller provided on the detection vehicle and connected to the pan and tilt and the infrared camera respectively, which is used to control capture actions of the infrared camera, to control angles of the horizontal and vertical rotations of the pan and tilt, and to transform the infrared thermal image digital signals output from the infrared camera into digital signals to be used in standard network transmission; and a data processor for generating and outputting control signals for the infrared camera, and to receive the digital infrared signals to be analyzed and processed to determine the types and locations of the defects on the detected road surface.

According to an embodiment of the present application, the main controller comprises: a USB signal transform board transforming the infrared thermal image digital signals contains the temperature values captured by the infrared camera into USB signals; a USB interface used to transmit the USB signals; and a data analysis and control module transforming the USB signals coming from the USB interface into the digital signals to be used in standard network transmission and then sending them in real time to the data processor through a network cable, generating control signals for the infrared camera according to distance measurement signals coming from the data processor, sending the control signals for the infrared camera to the infrared camera to control capture actions of the infrared camera, generating driving signals for the pan and tilt according to the control signals for the pan and tilt coming from the data processor, and sending the driving signals for the pan and tilt to the pan and tilt to control the horizontal and vertical rotations thereof.

According to an embodiment of the present application, the main controller is set in one protective hood together with the infrared camera.

According to an embodiment of the present application, the pan and tilt includes: a main base installed fixedly on the detection vehicle; a vertical drive motor set in the main base; a vertical rotary mechanism set on the main base and moving along with the vertical drive motor, so as to achieve the vertical rotation; a horizontal drive motor set in the main base; and a horizontal rotary mechanism set on the vertical rotary mechanism and moving along with the horizontal drive motor, so as to achieve the horizontal rotation, while the horizontal rotary mechanism carries out the vertical rotation along with the vertical rotary mechanism as to be driven by the vertical rotary mechanism; and a mounting bracket set on the horizontal rotary mechanism, which is used to detachably secure the body of the infrared camera.

According to an embodiment of the present application, the data processor include: a data input interface receiving the digital infrared signals from the main controller; a data analysis module using road surface defect analysis software to carry out image interception process and data analysis to the digital infrared signals coming from the data input interface, and to determine types and locations of the defects on the detected road surface based on the treated data; and a storage module recording therein the treated data and the determined results.

According to an embodiment of the present application, the data processor further comprises a display module for visually displaying infrared thermal images, digital infrared signals, defect types, defect locations and the proposed disposal solutions of the detected road surface. According to an embodiment of the present application, the data input interface of the data processor carries out data transmissions and network communications with the main controller via the standard RJ45 network interface.

According to an embodiment of the present application, the system further comprises: a distance measurement device set on a wheel of the detection vehicle, wherein the distance measurement device is connected to the data processor, receives speed pulse signals from the distance measurement device and converts the speed pulse signals into distance measurement signals, then outputs the distance measurement signals to the main controller, such that the main controller generates the control signals for the infrared camera according to the distance measurement signals to control capture actions of the infrared camera.

According to an embodiment of the present application, the system further comprises: a data transfer device connected to the main controller and the data processor respectively, so as to carry out network communications there between.

According to an embodiment of the present application, the data transfer device is a router to be connected to the main controller and the data processor via an internet cable.

To achieve the above object, the present application provides a method for road surface defects detection, which applies the system for road surface defects detection according to any one of claims 1-10 to detect the defects on the detected road surface, while the method comprising:
S1) the detection vehicle traveling on the detected road surface at an approximate uniform speed;
S2) the main controller generating driving signals for the pan and tilt to drive the horizontal and vertical rotations thereof, based on the control signals coming from the data processor;
S3) setting a predetermined interval based on the speed pulse signals coming from the distance measurement device;
S4) sending a trigger signal to the camera triggering means of the infrared camera at every other predetermined interval, wherein the infrared thermal imaging automatically captures the infrared thermal images of the detected road surface according to the trigger signal, and the infrared thermal images are infrared digital images with every spot of which containing a specific temperature value;
S5) the infrared camera sending each frame of the infrared thermal image digital signals containing the temperature values to the main controller in real-time via a data line, and the main controller transforming the infrared thermal image digital signals into digital signals to be used in standard network transmission and thus to be sent to the data processor;
S6) the data processor using road surface defect analysis software to carry out image interception process and data analysis to the digital infrared signals, to provide successive temperature distribution graphs for the detected road surface, and to obtain the temperature trending analysis curves to accurately reflect the capture timing for the detected road surface and the corresponding temperature of each spot thereof; and
S7) when the temperature data in the temperature distribution graphs show up obvious differences, determining types and locations of the defects on the detected road surface through the road surface defect analysis software.

According to an embodiment of the present application, the predetermined distance is 2 meters. According to an embodiment of the present application, the method further comprises: S8) after the implementation of steps S7, determining whether the detection vehicle reaches the end of the detected road surface, and returning to step S4 if the determined result is no.

According to an embodiment of the present application, the method further comprises: S9) after the implementation of step S8, if the determined result is yes, the analyst analyzing and judging all of the pictures showing up defects one by one to give conclusions of treatment and/or the proposed treatment solution, printing the malfunction report and filing it for backup, and the data processor also storing the images showing up defects and the relevant data into the database of the road defect analysis software.

According to an embodiment of the present application, in step S7, the data processor automatically notifying an alarm according to anomaly changed temperature distribution graphs and temperature curves.

According to an embodiment of the present application, in step S7, the data processor automatically converting position of the defects according to coordinates of the initial detected spot, the size of the predetermined interval, and the numbers and positions of the captured pictures showing up defects.

According to an embodiment of the present application, the method further comprises: S10) after the implementation of step S7, determining whether need to change the camera angle of the infrared camera, if the determined result is yes, returning to step S2, and if the determined result is no, then returning to step S4.

The technical solution of the present application has following beneficial effects:
①, In conjunction of the information collection technology such as infrared thermal imaging and the image digital infrared signal processing technology and the like, it's able to accomplish the rapid detection on road surface of the bituminous highway by using non-contact image captures. The system has excellent operation flexibility, clearly captured infrared images, and suffers no influence from bad weather conditions such as cloudy, rainy and foggy. Also, one single frame of image is captured only in 7 ms. As it's the thermal imaging detection, the details of the defects being captured can be shown more clearly, thus to determine on the defects more directly.
②, Triggered by the most advanced sensing technology, the system transfers signals in uniform distances or uniform intervals to the temperature monitoring device to secure the continuity of image captures.
③, The digital infrared signals are analyzed, processed and managed to generate an analysis report, thus it's able to discover hidden defects in the road surface in time, which improves the existing maintenance techniques for bituminous highway.

### BRIEF DESCRIPTION OF THE DRAWINGS

FIG. 1 is a block diagram showing the system for road surface defects detection according to an embodiment of the present application;
FIG. 2 is a schematic view showing the connection relationship between various parts in the system for road surface defects detection according to the embodiment of the present application in Fig. 1;
FIG. 3 is a flowchart showing the operation process of the system for road surface defects detection according to the embodiment of the present application;
FIG. 4 is a schematic view showing the structure of the pan and tilt according to an embodiment of the present application;
FIG. 5 is a schematic view showing the installed position of the system for road surface defects detection according to an embodiment of the present application;
FIG. 6 is a schematic view showing the installed position of the system for road surface defects detection according to another embodiment of the present application.

Wherein the reference signs are explained as below:
1-detection vehicle
2- pan and tilt
   21-main base
   22-verticle drive motor
   23-verticle rotary mechanism
   24-horizontal drive motor
   25-horizontal rotary mechanism
   26-fastness framework
3-infrared camera
4-main controller
   41-USB signal transform board
   42- USB interface
   43-data analysis and control module
5-data processor
   51-data input interface
   52-data analysis module
   53-storage module
   54-display module
6-distance measurement device
7-data transfer device

### DETAILED DESCRIPTION OF THE PREFERRED EMBODIMENTS

The foregoing and other objects, aspects and advantages of the present invention will become more apparent from the following detailed description of the present application when taken in conjunction with the preferred embodiments and accompanying drawings. The embodiments here are only used to illustrate but not to limit the present application.

Along with rapid developments of the highway and ceaseless increase of the built highway miles, it is paid more and more attention to the structural type selection, and the control to the construction technology and the construction quality is more and more strict, but some road sections may come forth damages such as water seepage, cracks, notches, wheel rut net crack, slurry jams, bridge floor break and so on after one or two year's traffic transport, which not only greatly reduce the highway's safety, fast, and comfortable traveling functions for usage, but also increase the cost of road maintenance.

In general, the damages on bituminous road surface can be divided into two types: one of which belongs to structural damages including damages on road surface structure as a whole or on any one or several parts of the road, such that the road surface can not withstand the vehicle load any more; and another one belongs to functional damages, which may not occur along with structural damages, but due to depressed factors of flatness as well as smoothness resistance, it is no longer qualified for some certain functions of usage, thus badly affecting the quality of traffic. Functional damages are generally superficial and easily identified, and their reasons of damages are relatively clear. The functional damages are mainly as follow: partial fine cracks, wave convexes, oil weeping, peeling, pockmarked surface, and wear and tear. Those damages mainly result in the depression of the service level of road, and generally have no direct relationship with the road surface structural characters. For the functional damages, surface functions can be restored by repairing, maintenance or layer overlaying.

Structural damages are mainly caused due to some of the intrinsic structural defects within layers of the road surface and the increased stress caused by loads, the results of which are reflected on the road surface showing up damages like cracks deformations and displacements in various shapes. Those damages are as following: chaps, net flaws, longitudinal and transverse cracks, subsidence, pits, slurry jams, after-repaired damages, looses and track ruptures. These damages not only lead to depression for the surface level of the road, but also apparently attenuate the structural bearing capacity of the road surface and increase the breakages, finally leading to the road surface to be ruined. As for the structural damages, the road often needs to be totally renovated.

In addition, the damage types of the road surface are often affected by the substrate types, surface layer structures, load types, climates, construction processes, and paved effects of the road. For example, in some bituminous surface layers of the broken-grade matched, rough and sliding off structure, there are often early watery damages such as water seepage and backwater and the like which are induced by relatively thick paved layer. In a region having climatic conditions of large temperature differences or low temperatures, horizontal or vertical cracks may appear on the bituminous road surface due to thermal stresses, and those cracks usually cause broken off and cataclasms and the like as affected by the vehicles.

On clearly knowing the damage types of the road, it's able to detect and cure the diseases of the road on pertinence. Since the current commonly used means of detection have defects of lower detection efficiency, narrow coverage, poor representation, high cost and destructive for the road surface, etc., it's necessary to continually develop the rapid, simple, effective and non-destructive road testing technology.

Infrared camera is widely used in the field of industrial or civil detection to capture infrared thermal images of the objects based on infrared technology, detect locations and work status of the objects, and from which to obtain the digital infrared signals of the captured objects.

Infrared camera is able to, by taking advantages of its working principle, capture images and as well to measure temperatures, which means that besides meeting the temperature measurement functions for spots of the infrared thermometer, the infrared camera can also present images showing the infrared thermal distribution for the detected object, and the accuracy digital infrared signals for each spot are also included in the images of the Infrared thermal distribution for the detected object, so that the test results are accurate and intuitive, therefore easy to carry out quick judgments to spots show up malfunctions or hidden troubles. Since infrared camera has optics mechanism similar to that of the camera, it can change the area range for an object to be detected by adjusting the angle of the lens' field of vision. If the infrared camera is mounted on top of the detection vehicle, it's enough to simultaneously detect all surfaces of four lanes of the highway. Therefore, using infrared camera to detect defects of rode surface has a very brilliant application prospect.

The system and method for road surface defects detection based on infrared imaging technology of the present invention is a new technology and new means able to meet above requirements for nondestructive detection on the road. By analyzing the formed infrared thermal images and then providing diagnosis of diseases on the road, it's able to carry out investigation and research on causes of disease or damages, and put forward practical and preventive maintenance solution, thereby to improve the quality of road construction and to extend the road's life-span, thus really putting in practice of "prevention first, prevention and treatments combined".

Infrared thermal Imaging technology mainly evaluate from both quantitative and qualitative aspects for the water seepage and damaged conditions of the road surface by collecting after rain temperatures on the surface of the bituminous highway, based on the infrared thermal image formed according to the temperature differences. Such as for chaps, net flaws and subsidence and other structural damages, because of the cracks and water seepages, there will be a certain temperature difference between the destroyed surface and the normal surface of the road, which is presented in the infrared thermal image as a low-temperature line in mesh or strip shape. The damage on the road surface can be briefly determined according to the temperature differences states, and then it's able to take appropriate repair methods on it. And for functional damages such as wave convexes, weeping, pockmarked surface and wear and tear etc., an analysis report is formed and then filed for backup by analyzing the differences data using an analysis software system. The analysis software is finally able to analyze types of the defects on road surface through accumulatively comparing the backup infrared thermal images.

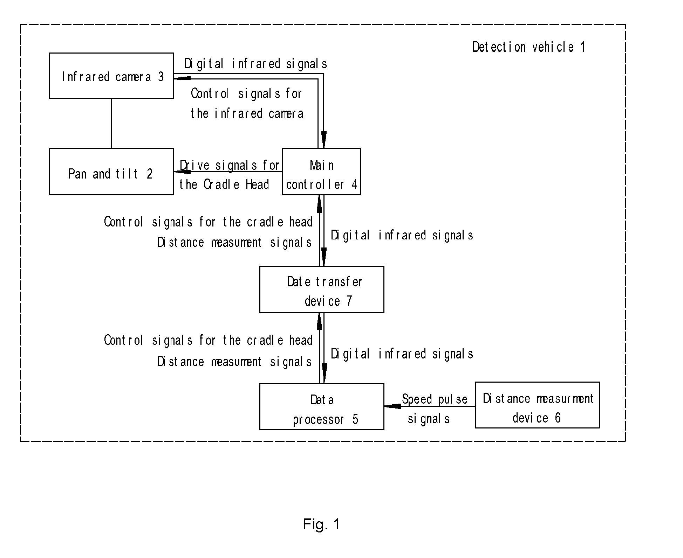

FIG. 1 is a block diagram showing the system for road surface defects detection according to an embodiment of the present application. As shown in Fig. 1, a system for road surface defects detection may comprise: a detection vehicle 1 traveling on a detected road surface in relatively uniform speed; a pan and tilt 2 provided on the detection vehicle 1 and rotate horizontally and vertically with respect to the detection vehicle 1; an infrared camera 3 detachablely set on the pan and tilt 2, which is used to capture infrared thermal images of the detected road surface, and to output inferred thermal image digital signals (14 bits digital signals) about the inferred thermal images including temperature values of the detected road surface; a main controller 4 provided on the detection vehicle 1 and connected to the pan and tilt 2 and the infrared camera 3 respectively, which is used to control capture actions (those capture actions includes turn-on timing, turn-off timing, trigger timing, focusing range and capture range, etc.) of the infrared camera 3, to control angles of the horizontal and vertical rotations of the pan and tilt 2, and to transform the infrared thermal image digital signals output from the infrared camera 3 into digital signals to be used in standard network transmission; and a data processor 5 for generating and outputting control signals for the infrared camera 3, and to receive the digital infrared signals to be analyzed and processed to determine the types and locations of the defects on the detected road surface.

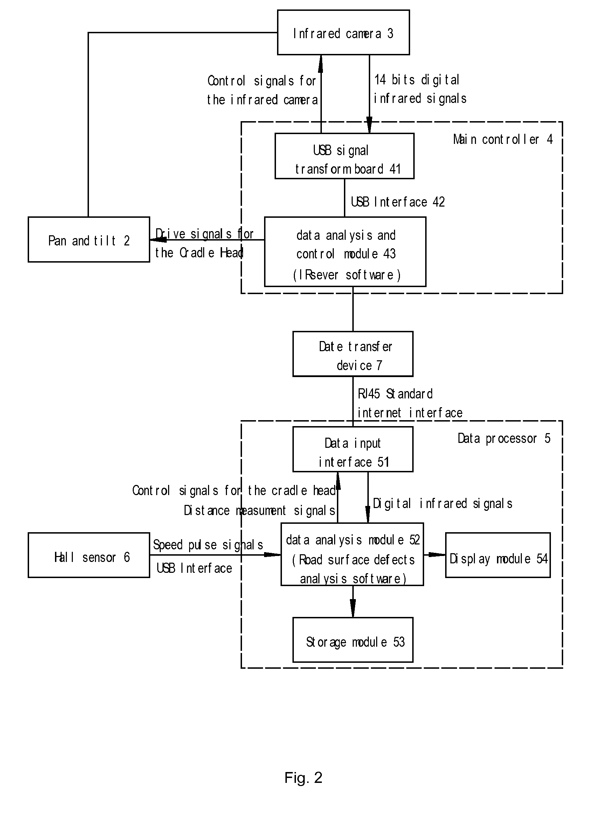

According to the embodiment shown in Fig. 2, the main controller 4 may comprise: a USB signal transform board 41 receiving and transforming the infrared thermal image digital signals containing the temperature values captured by the infrared camera 3 into USB signals; a USB interface 42 used to transmit the USB signals; and a data analysis and control module 43 transforming the USB signals coming from the USB interface 42 into the digital signals able to be used in RJ45 standard network interface transmission by using the IRsever software, and then sending the transformed digital signals in real time to the data processor 5 through a network cable, generating control signals for the infrared camera according to distance measurement signals coming from the data processor 5, sending the control signals for the infrared camera to the infrared camera 3 to control capture actions of the infrared camera 3, generating driving signals for the pan and tilt according to the control signals for the pan and tilt coming from the data processor 5, and sending the driving signals for the pan and tilt via an interface of 485 series to the pan and tilt 2 to control the horizontal and vertical rotations thereof.

For example, the main controller 4 may be implemented by the AMD LX700 chip, or may adopt any other kind of chips able to fulfill above described functions of the main controller 4. The main controller 4 is set in one protective hood together with the infrared camera 3.

Wherein the data analysis and control module 43 can transform the received 14 bits digital infrared signals (USB signals) coming from the infrared camera 3 into signals for RJ45 standard signals after operating the IRsever software, and then send those transformed signals to the data process 5 to deal with. The control signals (such as control signals of the infrared camera for core focusing and baffle rotation, and driving signals for the pan and tilt's rotation) are sent by the data processor 5 through RJ45 standard network interface to the main controller 4, and the main controller 4 may send USB signals after receiving the control signals and the driving signals, to drive the core imaging part of the infrared camera for a series of focusing actions and the like. The main controller 4 may also control the pan and tilt 2 via the 485 series to rotate horizontally and vertically within a certain range of angles.

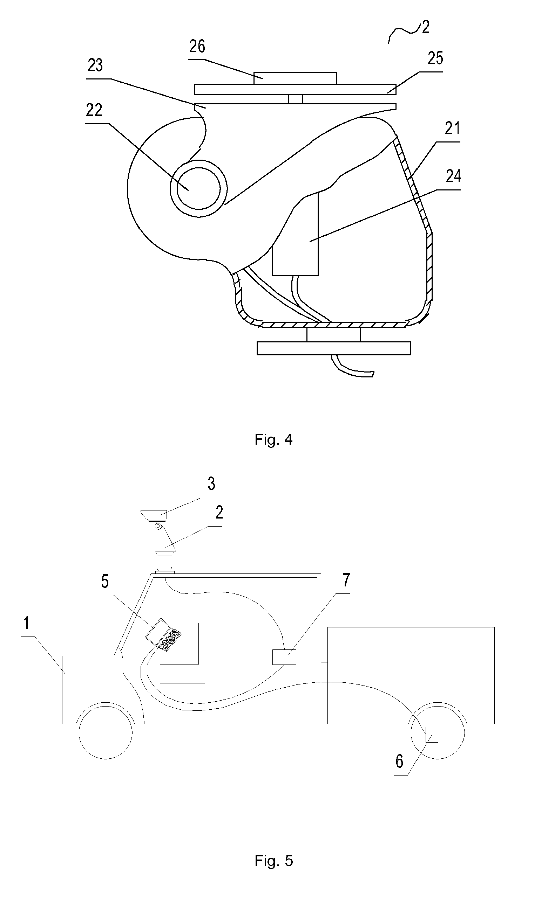

According to the embodiment shown in Fig. 4, the pan and tilt 2 may include: a main base 21 installed fixedly on the detection vehicle 1; a vertical drive motor 22 set in the main base 21; a vertical rotary mechanism 23 set on the main base 21 and moving along with the vertical drive motor 22, so as to achieve the vertical rotation; a horizontal drive motor 24 set in the main base 21; and a horizontal rotary mechanism 25 set on the vertical rotary mechanism 23 and moving along with the horizontal drive motor 23, so as to achieve the horizontal rotation, while the horizontal rotary mechanism 25 carries out the vertical rotation along with the vertical rotary mechanism 23 as to be driven by the vertical rotary mechanism 23 (the horizontal rotation at this time actually is not executed horizontally any more but obliquely); and a mounting bracket 26 set on the horizontal rotary mechanism 25, which is used to detachably secure the body of the infrared camera 3.

The vertical rotary mechanism 23 may have a rotation velocity of about 3-5 °/S, preferably 4°/S, and an angle of its vertical rotation is in a range of about 10-90°. The horizontal rotary mechanism 25 may have a rotation velocity of about 6-12 °/S, preferably 9°/S, and an angle of its horizontal rotation is in a range of about 0-355°.

The pan and tilt 2 may have functions of automatic scanning and automatic cruise.

The pan and tilt 2 may also has internet communication functions in a communication protocols of Pelco-P, Pelco-D, YAAN, and vocational criterion etc.

The pan and tilt 2 may also has a heater provided therein to warm up the environment within the main base 21 to ensure normal operations of all the parts in the pan and tilt 2 when the weather is too cold. The heater may be turned on for example at 8-±5°C, and turned off for example at 20-±5°C.

The data processor 5 may be implemented by a laptop computer which can be set within the detection vehicle or outside the detection vehicle at some where. For example, the laptop computer may be set in a workstation to remotely communicate with the data processor 5 and the main controller 4 via wireless internet.

The data processor 5 may include: a data input interface 51 receiving the digital infrared signals from the main controller 4; a data analysis module 52 using road surface defect analysis software to carry out image interception process and data analysis to the digital infrared signals coming from the data input interface 51, and to determine types and locations of the defects on the detected road surface based on the treated data; and a storage module 53 recording therein the treated data and the determined results.

The data processor 5 may further comprises a display module 54 for visually displaying the infrared thermal images, digital infrared signals, defect types, defect locations and the proposed disposal solutions for the detected road surface.

The data input interface 51 of the data processor 5 may be connected to the main controller 4 via the standard RJ45 network interface to carry out data transmissions and network communications with the main controller 4.

In one embodiment, the system of the present application may further comprise a distance measurement device 6 set on a wheel of the detection vehicle 1, wherein the distance measurement device 6 is connected to the data processor 5 to send speed pulse signals to the data processor 5, and the data processor 5 converts the speed pulse signals into distance measurement signals and then outputs the distance measurement signals to the main controller 4, such that the main controller 4 generates the control signals for the infrared camera according to the distance measurement signals to control capture actions of the infrared camera 3. Wherein the distance measurement device 6 may be for example a Hall sensor measuring the velocity of the wheels of the vehicle, or may be any other kind of distance measuring devices able to fulfill the requirements of the distance measuring functions of the vehicle.

Based on the pulse signals transmitted from the Hall sensor, the data processor 5 converts and calculates the detection vehicle's speed and stroke. The user needs to set parameters of the road surface defect analysis software in the data processor 5 according to the actual situation on the road to provide a predetermined interval (meter) for each time triggering the infrared camera 3 to capture infrared thermal images of the both sides of the road surface, each time when the vehicle travels a distance over the predetermined interval, the road surface defect analysis software automatically intercepts in the digital infrared signals sent from the main controller 4 and analyzes whether there is any surface defect character in the intercepted image, and, if there is any, automatically records the road section together with the surface defect character presented here to the storage module (the hard disk of the laptop computer) of the data processor 5 so as to be processed later.

In one embodiment, the system of the present application may further comprises a data transfer device 7 which may be a router connected to the main controller 4 and the data processor 5 respectively via an internet cable, so as to carry out data transmission and network communication between the main controller 4 and the data processor 5. Wherein the data transmission includes receiving the digital infrared signals output from the main controller 4 and sends them to the data processor 5, and receiving distance measurement signals and the control signals for the pan and tilt from the data processor 5 and send them to the main controller 4. The network communication includes the user directly sends control commands for the infrared camera and/or the control signals for the pan and tilt from the data processor 5 to the main controller 4.

In one embodiment, the system of the present application carries out data transmissions and network communications between the main controller 4 and data processor 5 by applying ISDN (Integrated Service Digital Network) technology using the RJ45 standard network interface.

As the system uses advanced computer compression technology and advanced network transmission technology, it's simply implemented by using one single network cable, through the standard RJ45 Ethernet interface, the network communication for the control signals between the main controller 4 and the data processor 5, the real time transmission, recordation and analysis processes for the Infrared images and the temperature data, and the automatic alarm notification for the malfunctions. The whole data (such as infrared thermal images, temperature data and control commands, etc.) are transmitted in digital format, thus greatly reducing the complexity in system wiring and the wiring cost as well, thus it's able to improve data transmission reliability, malfunction analysis accuracy and flexibility of the analysis and calculation on temperatures. The basic working principle of the system for surface defect detection of the present application is: during the detection vehicle's traveling, based on the obtained distance measurement signals from the distance measurement device 6 (Hall sensor) mounted on one of the wheels of the detection vehicle, a trigger signal is sent every time over a certain interval ( such as 2 meters) to trigger the infrared camera 3 to capture infrared thermal images with digital infrared signals for a certain sectional area (this area having a length of 2 meters and a width as a standard lane's width, for example), and then after the data analysis module 52 in the data processor 5 and the road surface defect analysis software therein analyzing on the infrared thermal images and the temperature data thereon, a temperature distribution graph for the road surface of the area is drawn based on the results of the analysis and process. By repeating the above operations, again and again, all of the continuous temperature distribution graphs and all the temperature data can be obtained for the whole detected road surface. The system of the present application can directly reflect the situation of defects on the detected road surface in ways of quantitative and qualitative methods, thus it's able to provide visual images and data for the road maintenance.

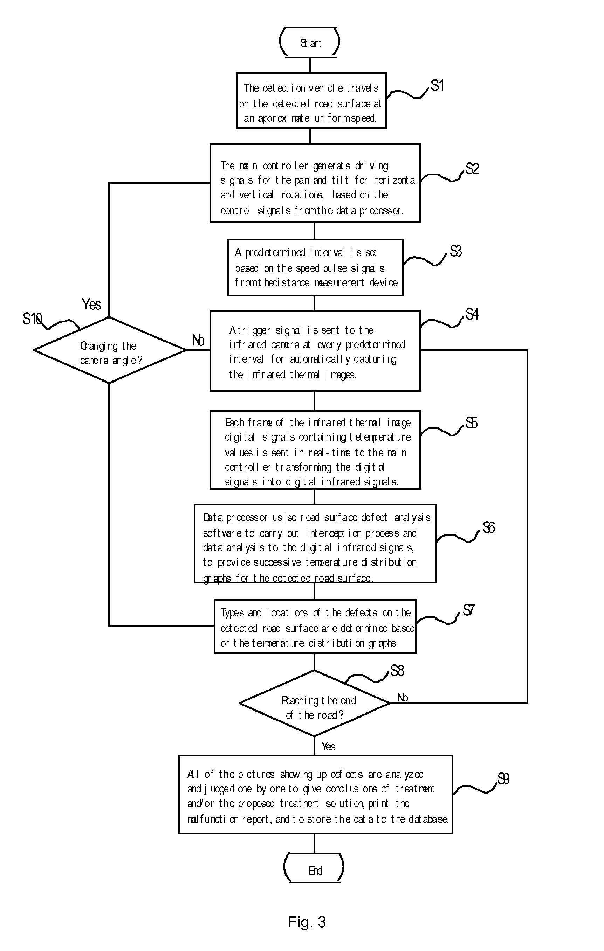

The method for road surface defects detection used by the system for road surface defects detection according to the embodiment of the present application is hereafter described with referring to Fig. 3.

As shown in Fig. 3, the method for road surface defects detection, which applies the system for road surface defects detection as above described to detect the defects on the detected road surface, comprises steps as below.

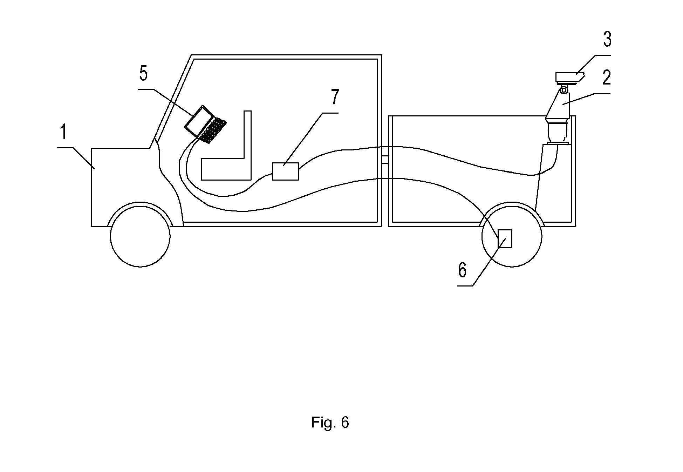

S1) The infrared camera 3 is mounted on a detection vehicle via a pan and tilt 2 (when taking into account the angle of the field of version taken by the infrared camera with the required height, the detection vehicle is usually implemented by a tool vehicle shown in Figs. 5-6 with its front head and rear portion having relatively higher distance away from the ground to facilitate the installation of the infrared camera). The main controller 4 and the Hall sensor are all connected through a network cable or a related power supply cable to the laptop computer (that is, the data processor 5) next to the driver's seat. The Installation location of the entire system is shown in Figs. 5-6.

Still referring to Figs. 5-6, the infrared camera 3 may be optionally mounted on the top or the rear portion of the detection vehicle according to specific road conditions and detection requirements. In installation, the infrared camera 3 can be elevated by an auxiliary plane to meet the requirements for detecting across the whole width of the road. Because of the different installed positions of the infrared camera 3, the calculation method on distance should take into account the installation location of the infrared camera 3 when determining the location of the defects according to the obtained infrared thermal images.

The detection vehicle 1 travels on the detected road surface at an approximate uniform speed.
S2) The main controller 4 generates driving signals for the pan and tilt for driving the horizontal and vertical rotations thereof, based on the control signals coming from the data processor 5.
S3) A predetermined interval (2 meters in general) is set based on the speed pulse signals coming from the Hall sensor.
S4) A trigger signal is sent to the camera triggering means of the infrared camera 3 at every other predetermined interval, the infrared thermal imaging 3 automatically captures the infrared thermal images of the detected road surface according to the trigger signal, wherein the infrared thermal images are infrared digital images with every spot of which containing a specific temperature value.
S5) The infrared camera 3 sends each frame of the infrared thermal image digital signals containing the temperature values to the main controller 4 in real-time via a data line, and the main controller 4 transforms the infrared thermal image digital signals into digital signals to be used in standard network transmission and thus to be sent to the data processor 5.
S6) The data processor 5 uses road surface defect analysis software to carry out image interception process and data analysis to the digital infrared signals, to provide successive temperature distribution graphs for the detected road surface, and to obtain the temperature trending analysis curves to accurately reflect the capture timing for the detected road surface and the corresponding temperature of each spot thereof.
S7) When the temperature data in the temperature distribution graphs show up obvious differences, the road surface defect analysis software may analyze those data differences and then figure out an analysis report to determine types of the defects on the detected road surface. The road surface defect analysis software may automatically notify an alarm for the abnormal changed temperature images and curves such as by automatically pop-out an alarm prompt window on the display screen of the display module, and tell the numbers and positions of the captured pictures showing up defects based on the coordinates of the initial detected spot. The computer process system then automatically converts and calculates the specific locations of the defects on the detected road surface (which is the miles the defect is posited form the initial detected spot) according to the numbers and positions together with the parameters of the triggering interval predetermined by the distance measurement device (the Hall sensor).

The method for road surface defects detection further comprises steps as below,
S8) After the implementation of steps S7, it is determined that the detection vehicle 1 whether or not reaches the end of the detected road surface, returning to step S4 if the determined result is no.
S9) After the implementation of step S8, if the determined result is yes, the analyst analyzes and judges all of the pictures showing up defects one by one to give conclusions of treatment and/or the proposed treatment solutions, and to print and file the malfunction report for backup, and the data processor 5 also records the images showing up defects and the relevant data into the database of the road defect analysis software for accumulation and summarization usages in order to guide the afterward repair and maintenance works better.
S10) After the implementation of step S7, it's determined whether need to change the camera angle of the infrared camera 3, if the determined result is yes, returning to step S2, and if the determined result is no, returning to step S4.

Compared with the prior art, the system and method for surface defect detection of the present application based on infrared imaging technology has following advantages:
a) The existing infrared thermometer is unable to capture images but only measure the spot temperatures, while the system and the method of the present application is able to not only measure temperatures but also display the infrared thermal images of the detected objects, and the detection results of which is relatively more intuitive, fast, accurate and reliable to the prior art, thus providing effective basis for the following malfunction diagnosis and expelling operations, which plays multiplier effects with less efforts.
b) As the infrared thermometer in the prior art actually detects the average temperature of a piece of area of the detected object surface (so-called measured area, the size of which is in proportional to the distance of temperature measuring), so that the measured temperatures may have certain errors in accuracy. While the present application first of all is able to display an infrared thermal distribution graph of the whole detected road surfaces, and obtain specific temperature values for every spot on the infrared thermal distribution graph simultaneously, resulting in more intuitive and accurate detection results, and thus it's able to provide intuitive and reliable basis for determining the defect points and as well as for the post-processing operations which is unavailable for the prior art.

As the infrared thermometer in the prior art actually only detect the average temperature of a piece of the measured area of the detected object surface (the size of which is in proportional to the distance of temperature measuring), so that the closer the infrared thermometer is to the road surface, the smaller the size of the measured area is, and the further the infrared thermometer is from the road surface, the bigger the size of the measured area is. However, as the measured temperature of the infrared thermometer needs to meet certain accuracy requirements, in order to obtain accurate temperature measurements, the infrared thermometer shall not be installed at a location too far away from the road. Thus, the area to be detected by one single infrared thermometer each time is extremely limited, and even if more than one infrared thermometer are mounted in a detection vehicle, it's still not easy to monitor more than one lane once at a time, and thus the detection on a multi-lane road is rather time-consuming and difficult. However, the system of the present application can adjust its field of version of the camera to transform the measured range of the detected object, it's easy to achieve simultaneously detection for a highway having, for example, 4 lanes, thus greatly improving the detection efficiency and accuracy which is unavailable for the prior art.

In the above embodiment, the system of the present application applies the infrared imaging technology to the defects detection on the bituminous road surface or other surfaces of the already built highways, but the embodiments of the present application are not limited to this. The infrared imaging technology can also be applied to a constructing site of a road still in construction, so as to provide infrared images and related data for references on uniformity of bitumen paving in the construction operations of the bituminous road surface of the highway and the like, thus to ensure the construction quality.

Although embodiments have been described with reference to a number of illustrative embodiments thereof, it should be understood that numerous other modifications and embodiments can be devised by those skilled in the art that will fall within the spirit and scope of the principles of embodiments. More particularly, various variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. A system for road surface defects detection, comprising:
a detection vehicle (1) traveling on a detected road surface;
a pan and tilt (2) provided on the detection vehicle (1) and rotate horizontally and vertically with respect to the detection vehicle (1);
an infrared camera (3) detachably set on the pan and tilt (2), which is used to capture infrared thermal images of the detected road surface, and to output inferred thermal image digital signals about the inferred thermal images including temperature values of the detected road surface;
a main controller (4) provided on the detection vehicle (1) and connected to the pan and tilt (2) and the infrared camera (3) respectively, which is used to control capture actions of the infrared camera (3), to control angles of the horizontal and vertical rotations of the pan and tilt (2), and to transform the infrared thermal image digital signals output from the infrared camera (3) into digital signals to be used in standard network transmission; and
a data processor (5) for generating and outputting control signals for the infrared camera (3), and
to receive the digital infrared signals to be analyzed and processed to determine the types and
locations of the defects on the detected road surface.

2. The system for road surface defects detection according to claim 1, wherein the main controller (4) comprises:
a USB signal transform board (41) transforming the infrared thermal image digital signals contains the temperature values captured by the infrared camera (3) into USB signals;
a USB interface (42) used to transmit the USB signals; and
a data analysis and control module (43) transforming the USB signals coming from the USB interface (42) into the digital signals to be used in standard network transmission and then sending them in real time to the data processor (5) through a network cable, generating control signals for the infrared camera according to distance measurement signals coming from the data processor (5), sending the control signals for the infrared camera to the infrared camera (3) to control capture actions of the infrared camera (3), generating driving signals for the pan and tilt according to the control signals for the pan and tilt coming from the data processor (5), and sending the driving signals for the pan and tilt to the pan and tilt (2) to control the horizontal and
vertical rotations thereof.

3. The system for road surface defects detection according to claim 2, wherein the main controller (4) is set in one protective hood together with the infrared camera (3).

4. The system for road surface defects detection according to claim 1, wherein the pan and tilt (2) includes:
a main base (21) installed fixedly on the detection vehicle (1);
a vertical drive motor (22) set in the main base (21);
a vertical rotary mechanism (23) set on the main base (21) and moving along with the vertical drive motor (22), so as to achieve the vertical rotation;
a horizontal drive motor (24) set in the main base (21); and
a horizontal rotary mechanism (25) set on the vertical rotary mechanism (23) and moving along with the horizontal drive motor (23), so as to achieve the horizontal rotation, while the horizontal rotary mechanism (25) carries out the vertical rotation along with the vertical rotary mechanism (23 ) as to be driven by the vertical rotary mechanism (23 ); and
a mounting bracket (26) set on the horizontal rotary mechanism (25), which is used to detachably secure the body of the infrared camera (3).

5. The system for road surface defects detection according to claim 1, wherein the data processor (5) include:
a data input interface (51) receiving the digital infrared signals from the main controller (4);
a data analysis module (52) using road surface defect analysis software to carry out image interception process and data analysis to the digital infrared signals coming from the data input interface (51), and to determine types and locations of the defects on the detected road surface based on the treated data; and
a storage module (53) recording therein the treated data and the determined results.

6. The system for road surface defects detection according to claim 5, wherein:
• the data processor (5) further comprises a display module (54) for visually displaying infrared thermal images, digital infrared signals, defect types, defect locations and the proposed disposal solutions of the detected road surface; or
• the data input interface (51) of the data processor (5) carries out data transmissions and network communications with the main controller (4) via the standard RJ45 network interface.

7. The system for road surface defects detection according to claim 1, wherein the system further comprises:
a distance measurement device (6) set on a wheel of the detection vehicle (1), wherein the distance measurement device (6) is connected to the data processor (5), receives speed pulse signals from the distance measurement device (6) and converts the speed pulse signals into distance measurement signals, then outputs the distance measurement signals to the main controller (4), such that the main controller (4) generates the control signals for the infrared camera according to the distance measurement signals to control capture actions of the infrared camera (3).

8. The system for road surface defects detection according to claim 1, wherein the system further comprises:
a data transfer device (7) connected to the main controller (4) and the data processor (5) respectively, so as to carry out network communications therebetween; preferably the data transfer device (7) is a router to be connected to the main controller (4) and the data processor (5) via an internet cable.

9. A method for road surface defects detection, which applies the system for road surface defects detection according to any one of claims 1-10 to detect the defects on the detected road surface, while the method comprising:
S1) the detection vehicle (1) traveling on the detected road surface at an approximate uniform speed;
S2) the main controller (4) generating driving signals for the pan and tilt to drive the horizontal and vertical rotations thereof, based on the control signals coming from the data processor (5);
S3) setting a predetermined interval based on the speed pulse signals coming from the distance measurement device (6);
S4) sending a trigger signal to the camera triggering means of the infrared camera (3) at every other predetermined interval, wherein the infrared thermal imaging (3) automatically captures the infrared thermal images of the detected road surface according to the trigger signal, wherein the infrared thermal images are infrared digital images with every spot of which containing a specific temperature value;
S5) the infrared camera (3) sending each frame of the infrared thermal image digital signals containing the temperature values to the main controller (4) in real-time via a data line, and the main controller (4) transforming the infrared thermal image digital signals into digital signals to be used in standard network transmission and thus to be sent to the data processor (5);
S6) the data processor (5) using road surface defect analysis software to carry out image interception process and data analysis to the digital infrared signals, to provide successive temperature distribution graphs for the detected road surface, and to obtain the temperature trending analysis curves to accurately reflect the capture timing for the detected road surface and the corresponding temperature of each spot thereof; and
S7) when the temperature data in the temperature distribution graphs show up obvious differences, determining types and locations of the defects on the detected road surface through the road surface defect analysis software.

10. The method for road surface defects detection according to claim 9, wherein the predetermined distance is 2 meters.

11. The method for road surface defects detection according to claim 9, the method further comprises:
S8) after the implementation of steps S7, determining whether the detection vehicle (1) reaches the end of the detected road surface, and returning to step S4 if the determined result is no.

12. The method for road surface defects detection according to claim 11, the method further comprises:
S9) after the implementation of step S8, if the determined result is yes, the analyst analyzing and judging all of the pictures showing up defects one by one to give conclusions of treatment and/or the proposed treatment solution, and print the malfunction report and file it for backup, and the data processor (5) storing the images showing up defects and the relevant data into the database of the road defect analysis software.

13. The method for road surface defects detection according to claim 9, wherein, in step S7, the data processor (5) automatically notifying an alarm according to anomaly changed temperature distribution graphs and temperature curves.

14. The method for road surface defects detection according to claim 9, wherein, in step S7, the data processor (5) automatically converting position of the defects according to coordinates of the initial detected spot, the size of the predetermined interval, and the numbers and positions of the captured pictures showing up defects.

15. The method for road surface defects detection according to claim 9, wherein the method further comprises:
S10) after the implementation of step S7, determining whether need to change the camera angle of the infrared camera (3) , if the determined result is yes, returning to step S2, and if the determined result is no, then returning to step S4.
